# EUROPEAN PATENT APPLICATION

(11) **EP 2 779 262 A1**
(43) Date of publication of application: **17.09.2014**
(21) Application number: 12846834.5
(22) Date of filing: 06.11.2012
(51) Int. Cl.: H01L 51/42, C07C 13/64, C07C 49/92

(54) **PHOTOVOLTAIC CONVERSION ELEMENT AND METHOD OF MANUFACTURE THEREOF**

(30) Priority: 07.11.2011 JP 2011243896
(71) Applicant: JX Nippon Oil & Energy Corporation, Chiyoda-ku Tokyo 100-8162 (JP)
(72) Inventor: ICHIBAYASHI, Taku, Chiyoda-ku, Tokyo 100-8162 (JP); ASANO, Tsuyoshi, Chiyoda-ku, Tokyo 100-8162 (JP)
(74) Representative: Algemeen Octrooi- en Merkenbureau B.V.
(86) International application number: PCT/JP2012/007114
(87) International publication number: WO 2013/069261

(57) **Abstract**

A photoelectric conversion element 10 has a structure in which an electron transport layer 40, a conduction band bottom energy adjustment layer 50, a photoelectric conversion layer 60, and a hole transport layer 70 are sandwiched in this order between a first electrode 30 and a second electrode 80. The conduction band bottom energy adjustment layer 50 is formed, for example, of cesium carbonate. The electron transport layer 40 is formed of zinc acetate.

## Description

### TECHNICAL FIELD

The present invention relates to a photoelectric conversion element configured to convert light energy into electric energy by photoelectric conversion.

### BACKGROUND ART

Organic thin-film solar cells (photoelectric conversion elements) using an organic semiconductor can be manufactured by simple techniques, such as printing, that is, by processes suitable for manufacturing solar cells having a large area and for mass production, such as a roll-to-roll process, and by using lightweight and flexible materials, such as a plastic substrate, and accordingly they have the futures that they are more inexpensive, lightweight, and flexible than conventional ones. Therefore, they are expected to be used in an extended range of applications and are considered as a promising next-generation solar cell. The organic thin-film solar cells are currently being studied such that the photoelectric conversion efficiencies thereof are improved toward practical use thereof, and solar cells having a photoelectric conversion efficiency of 8% or more have been developed. However, it is known that the organic thin-film solar cells are deteriorated due to light, heat, and oxygen and moisture in the air, and accordingly it is extremely important to improve the durability of an element for practical use.

In a typical configuration of an organic thin-film solar cell, it is normal to form a good ohmic contact between an electrode and a photoelectric conversion layer and to provide a charge extraction layer between them in order to efficiently extract charges generated in the charge extraction layer. Until now, the case where an organic compound, such as 2,9-dimethyl- 4,7-diphenyl-1,10-phenanthroline (BCP), phosphine oxide compound, or the like, is used in an electron extraction layer, one type of the charge extraction layer, has been reported (Non-Patent Document 1 and Patent Document 1), and the case where an inorganic compound, such as lithium fluoride (LiF), titanium oxide (TiOₓ), zinc oxide (ZnO), cesium carbonate (Cs₂CO₃), or the like, is used therein has been reported (Non-Patent Documents 2 to 5). As an example in which BCP is used in an electron extraction layer made of an organic compound material, a low molecule vapor deposition organic thin-film solar cell, in which pentacene and fullerene C60 are combined, has been reported in Non-Patent Document 1. However, there remains the problem that a photoelectric conversion efficiency is decreased due to light emission for a short period of time, and accordingly a further improvement is required.

Also, a low molecule vapor deposition organic thin-film solar cell using a phosphine oxide compound has been reported in Patent Document 1. However, the photoelectric conversion efficiency is low, and accordingly it is required to improve the efficiency in order to put it in practical use. As an example in which LiF is used in an electron extraction layer made of an inorganic compound material, a high molecule application organic thin-film solar cell, in which a polymer semiconductor (MEH-PPV) and a fullerene derivative (PCBM) are combined, has been reported in Non-Patent Document 2. However, there remains the problem that the photoelectric conversion efficiency is low and the efficiency is decreased due to light emission for a short period of time, and accordingly it is required to improve the efficiency and durability in order to put it in practical use. As an example using TiOₓ, a high molecule application organic thin-film solar cell, in which a polymer semiconductor (P3HT) and a fullerene derivative (PCBM) are combined, has been reported in Non-Patent Document 3, which says that it has excellent durability. However, the durability, obtained after light emission for a relatively short period of time of approximately 20 hours, has only been reported, and accordingly it is not sufficient for practical use. As an example using ZnO or Cs₂CO₃, a high molecule application organic thin-film solar cell, in which a polymer semiconductor (P3HT) and a fullerene derivative (PCBM) are combined, has been reported in Non-Patent Documents 4 and 5; however, the photoelectric conversion efficiency and durability are both insufficient.

### [Related Art Documents]

### [Patent Documents]

[Patent Document 1] Japanese Patent Application Publication No. 2006-073583
[Patent Document 2] Japanese Patent Application Publication No. 2011-119648

### [Non-Patent Documents]

[Non-Patent Document 1] Organic Electronics 2008, Vol. 9, p. 656-660
[Non-Patent Document 2] Sol. Energy Mater. Sol. Cells 2005, Vol. 86, p. 499-516
[Non-Patent Document 3] Sol. Energy Mater. Sol. Cells 2008, Vol. 92, p. 1476-1482
[Non-Patent Document 4] Sol. Energy Mater. Sol. Cells 2011, Vol. 95, p. 1382-1388
[Non-Patent Document 5] Sol. Energy Mater. Sol. Cells 2010, Vol. 94, p. 1831-1834

### DISCLOSURE OF THE INVENTION

Problem to be Solved by the Invention as described above, in a conventional organic thin-film solar cell, a photoelectric conversion efficiency is remarkably decreased due to light emission for a long period of time, which becomes a constraint for the long-life of the solar cell. Accordingly, there is a need for a solar cell having a sufficiently high photoelectric conversion efficiency, not only in the initial state where sunlight is not emitted, but also in a state where sunlight is emitted for a long period of time.

The present invention has been made in view of these problems, and a purpose of the invention is to provide a technique in which the photoelectric conversion efficiency of a photoelectric conversion element can be improved in a state where sunlight is emitted for a long period of time.

### Means for Solving the Problem

An aspect of the present invention is a photoelectric conversion element. The photoelectric conversion element comprises: at least a photoelectric conversion layer; an electron extraction electrode provided on one major surface side of the photoelectric conversion layer; a hole extraction electrode provided on the other major surface side of the photoelectric conversion layer; and an electron extraction layer that is provided between the photoelectric conversion layer and the electron extraction electrode and includes at least an electron transport layer, in which the photoelectric conversion element further comprises, between the photoelectric conversion layer and the electron transport layer, a conduction band bottom energy adjustment layer configured to reduce conduction band bottom energy of the electron extraction layer to energy lower than conduction band bottom energy of the electron transport layer.

According to a photoelectric conversion element of this aspect, a photoelectric conversion efficiency in the initial state can be improved, and a decrease in the photoelectric conversion efficiency, which may occur in a state where light is continuously emitted for a long period of time, can be suppressed. Thereby, the life of the photoelectric conversion element can be extended.

In a photoelectric conversion element of the aforementioned aspect, when xenon lamp light, the ultraviolet ray intensity of which is adjusted to be equivalent to 1 Sun, is continuously emitted at ambient temperature of 40°C for 1000 hours from the initial state where the light is not emitted, a decrease rate of the photoelectric conversion efficiency may be 10% or less. The conduction band bottom energy adjustment layer may contain a cesium compound.

Also, the electron transport layer may contain a substance represented by the following chemical formula or a reactant obtained from one or more substances represented by the following chemical formula:

M(X)ₐ (1)

wherein M is a metal or alloy selected from the group consisting of alkali metals, alkaline earth metals, group 2B and 3B metals, and transition metals; X is selected from oxygen, a halogen, carboxylate group, alkoxy group, alkyl group, and acetonate group represented by the following formula; and a is a positive integer determined in accordance with the valence of M: 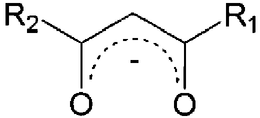 wherein R₁ and R₂ are selected from hydrogen, a C₁₋₂₀ linear or branched alkyl group, and C₁₋₂₀ linear or branched alkoxy group, and R₁ and R₂ may or may not be the same as each other.

The electron transport layer may include one or more metal compounds and reactants thereof, the metal compounds being selected from the group consisting of zinc acetate, magnesium acetate, aluminum acetylacetonate, aluminum chloride, gallium acetylacetonate, gallium chloride, zinc acetylacetonate, zinc chloride, diethylzinc, and ZnMgO. The photoelectric conversion layer may contain a fullerene derivative having a first reduction potential of 1160 mV (vs Fc/Fc⁺) or more. The fullerene derivative may be ICBA (Indene-C60 bisadduct).

Another aspect of the present invention is a method of manufacturing a photoelectric conversion element having a pair of electrodes, a photoelectric conversion layer provided between the pair of electrodes, an electron transport layer provided between one of the electrodes and the photoelectric conversion layer, and a conduction band bottom energy adjustment layer interposed between the photoelectric conversion layer and the electron transport layer, the method comprising: forming the electron transport layer by heating a film, which has been formed by applying a solution containing a substance represented by the following chemical formula (2), to a temperature (t₁) of 100°C ≤ t₁ ≤ 150°C; and forming the conduction band bottom energy adjustment layer containing a cesium compound:

Zn(X)₂ (2)

wherein X is selected from oxygen, a halogen, carboxylate group, alkoxy group, alkyl group, and acetonate group represented by the following formula: 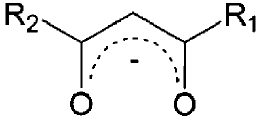 wherein R₁ and R₂ are selected from hydrogen, a C₁₋₂₀ linear or branched alkyl group, and C₁₋₂₀ linear or branched alkoxy group, and R₁ and R₂ may or may not be the same as each other.

Still another embodiment of the present invention is a method of manufacturing a photoelectric conversion element having a pair of electrodes, a photoelectric conversion layer provided between the pair of electrodes, an electron transport layer provided between one of the electrodes and the photoelectric conversion layer, and a conduction band bottom energy adjustment layer interposed between the photoelectric conversion layer and the electron transport layer, the method comprising: forming the electron transport layer by heating a film, which has been formed by applying a solution containing substances respectively represented by the following chemical formulae (1-1) and (1-2), to a temperature of 300°C or higher; and forming the conduction band bottom energy adjustment layer containing a cesium compound:

Zn(X)₂ (1-1)

wherein X is selected from oxygen, a halogen, carboxylate group, alkoxy group, alkyl group, and acetonate group represented by the following formula: 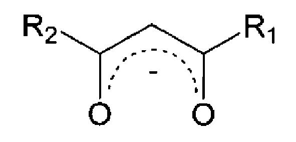 wherein R₁ and R₂ are selected from hydrogen, a C₁₋₂₀ linear or branched alkyl group, and C₁₋₂₀ linear or branched alkoxy group, and R₁ and R₂ may or may not be the same as each other,

Mg(X)₂ (1-2)

wherein X is selected from oxygen, a halogen, carboxylate group, alkoxy group, alkyl group, and acetonate group represented by the following formula: 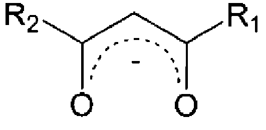 wherein R₁ and R₂ are selected from hydrogen, a C₁₋₂₀ linear or branched alkyl group, and C₁₋₂₀ linear or branched alkoxy group, and R₁ and R₂ may or may not be the same as each other.

An aspect in which the respective components described above are appropriately combined can be encompassed within the scope of the invention for which protection is sought by this application.

### Advantage of the Invention

According to the present invention, the photoelectric conversion efficiency of a photoelectric conversion element can be improved in a state where sunlight is emitted for a long period of time.

### BRIEF DESCRIPTION OF THE DRAWINGS

Fig. 1 is a schematic sectional view illustrating a structure of a photoelectric conversion element according to an embodiment;
Fig. 2 is a graph showing light resistance of each of the photoelectric conversion elements of Examples 1 to 3 and Comparative Examples 1 to 6; and
Fig. 3 is a graph showing light resistance of each of the photoelectric conversion elements of Examples 4 to 6 and Comparative Examples 7 to 12.

### MODE FOR CARRYING OUT THE INVENTION

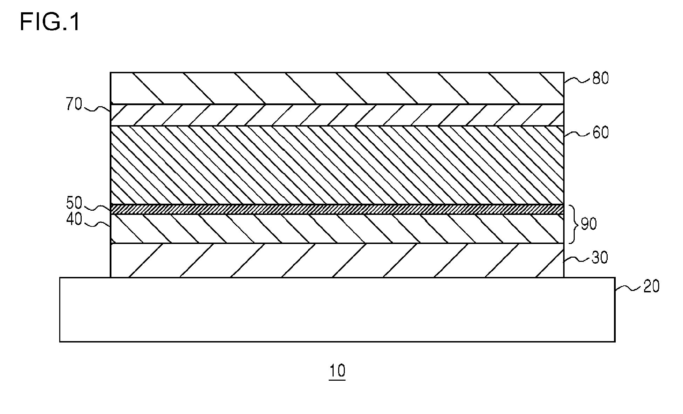

Hereinafter, embodiments of the present invention will be described with reference to the accompanying drawings. Fig. 1 is a schematic sectional view illustrating a structure of a photoelectric conversion element 10 according to an embodiment. The photoelectric conversion element 10 of the present embodiment is an organic thin-film solar cell that includes a photoelectric conversion layer containing an organic semiconductor.

The photoelectric conversion element 10 according to the present embodiment comprises a substrate 20, a first electrode 30, an electron transport layer 40, a conduction band bottom energy adjustment layer 50, a photoelectric conversion layer 60, a hole transport layer 70, and a second electrode 80. In the following description, a layer including at least the electron transport layer 40 and the conduction band bottom energy adjustment layer 50 is referred to as an electron extraction layer 90.

In the present embodiment, the first electrode 30 is a negative electrode (electron extraction electrode) and is electrically connected to the later-described photoelectric conversion layer 60 via the electron extraction layer 90. The first electrode 30 is located on the light-receiving surface side of the photoelectric conversion layer 60, and is formed of: a conductive metal oxide, such as ITO (Indium Tin Oxide), SnO₂, FTO (Fluorine doped Tin Oxide), ZnO, AZO (Aluminum doped Zinc Oxide), IZO (Indium doped Zinc Oxide), or the like; a metal thin film, such as gold, silver, copper, aluminum, or the like; or a transparent conducting film, such as a mesh, a stripe, or the like. The first electrode 30 is formed on the light-transmissive substrate 20 so as not to inhibit a light-receiving performance. The substrate 20 may be formed, for example, of colorless or colored glass, wire glass, a glass block, or the like; or a colorless or colored transparent resin. Specific examples of such a resin include polyester, such as polyethylene terephthalate, polyamide, polysulfone, polyether sulfone, polyether ether ketone, polyphenylene sulfide, polycarbonate, polyimide, polymethylmethacrylate, polystyrene, tri-cellulose acetate, and polymethyl pentene, etc.

The electron extraction layer 90 is formed in a region between the first electrode 30 and the photoelectric conversion layer 60 and includes at least the electron transport layer 40 and the conduction band bottom energy adjustment layer 50. The electron transport layer 40 functions to facilitate the transport of electrons from the photoelectric conversion layer 60 to the first electrode 30. The electron transport layer 40 can also function such that the transport of holes from the photoelectric conversion layer 60 to the first electrode 30 is hardly caused. The thickness of the electron transport layer 40 is not particularly limited, but it is, for example, 10 to 100 nm, and preferably 20 to 60 nm.

The electron transport layer 40 contains a substance represented by the following chemical formula and a reactant obtained from one or more substances represented by the following chemical formula:

M(X)ₐ (1)

wherein M is selected from the group consisting of alkali metals, alkaline earth metals, group 2B and 3B metals, and transition metals; X is selected from a halogen, carboxylate group, alkoxy group, alkyl group, and acetonate group represented by the following formula; and a is a positive integer determined in accordance with the valence of M: 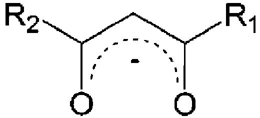 wherein R₁ and R₂ are selected from hydrogen, a C₁₋₂₀ linear or branched alkyl group, and C₁₋₂₀ linear or branched alkoxy group, and R₁ and R₂ may or may not be the same as each other. Examples of the alkyl group include, for example, a methyl group, ethyl group, and propyl group, etc.; and examples of the alkoxy group include, for example, a methoxy group and ethoxy group, etc.

Specific examples of M include Li, Na, Mg, Al, K, Ca, Sc, Ti, V, Cr, Mn, Fe, Co, Ni, Cu, Zn, Ga, Rb, Sr, Zr, Mo, Ru, Rh, Pd, Ag, Cd, In, Sn, Cs, Ba, La, Ir, Pt, Hg, Tl, Pb, ans Bi, etc. Specific examples of X include: ions of halogens, such as fluorine, chlorine, bromine, and iodine, etc.; carboxylate groups derived from carboxylic acids, such as formic acid, acetic acid, propionic acid, butyric acid, oxalic acid, malonic acid, succinic acid, glutaric acid, phthalic acid, acrylic acid, methacrylic acid, citric acid, ethylenediaminetetraacetate, and benzoic acid, etc. The alkoxy group to be used in X is not particularly limited, but C₁₋₁₀ linear or branched alkoxy groups, such as, for example, a methoxy group, ethoxy group, and propoxy group, can be cited. The alkyl group to be used in X is not particularly limited, but C₁₋₁₀ linear or branched alkyl groups, such as a methyl group, ethyl group, and propyl group, can be cited.

More specific examples of the substance to be contained in the electron transport layer 40 include lithium fluoride, lithium chloride, lithium iodide, magnesium chloride, zinc chloride, aluminum chloride, gallium chloride, nickel chloride, gallium chloride, lithium formate, sodium formate, magnesium formate, potassium formate, calcium formate, manganese formate, nickel formate, copper formate, zinc formate, rubidium formate, strontium formate, cesium formate, barium formate, thallium formate, lead formate, lithium acetate, sodium acetate, magnesium acetate, aluminum acetate, potassium acetate, calcium acetate, chromium acetate, manganese acetate, iron acetate, cobalt acetate, nickel acetate, copper acetate, zinc acetate, rubidium acetate, strontium acetate, zirconium acetate, molybdenum acetate, rhodium acetate, palladium acetate, silver acetate, cadmium acetate, indium acetate, tin acetate, cesium acetate, barium acetate, mercury acetate, thallium acetate, lead acetate, bismuth acetate, lithium propionate, sodium propionate, magnesium propionate, potassium propionate, calcium propionate, manganese propionate, nickel propionate, zinc propionate, strontium propionate, palladium propionate, barium propionate, lead propionate, lithium butyrate, sodium butyrate, magnesium butyrate, potassium butyrate, calcium butyrate, manganese butyrate, nickel butyrate, zinc butyrate, strontium butyrate, barium butyrate, lead butyrate, aluminum acetylacetonate, scandium acetylacetonate, vanadium acetylacetonate, chromium acetylacetonate, manganese acetylacetonate, iron acetylacetonate, cobalt acetylacetonate, nickel acetylacetonate, copper acetylacetonate, zinc acetylacetonate, gallium acetylacetonate, zirconium acetylacetonate, ruthenium acetylacetonate, rhodium acetylacetonate, palladium acetylacetonate, silver acetylacetonate, indium acetylacetonate, lanthanum acetylacetonate, neodymium acetylacetonate, samarium acetylacetonate, europium acetylacetonate, gadolinium acetylacetonate, terbium acetylacetonate, erbium acetylacetonate, iridium acetylacetonate, platinum acetylacetonate, thallium acetylacetonate, lead acetylacetonate, sodium oxalate, sodium hydrogen oxalate, potassium oxalate, potassium hydrogen oxalate, lithium oxalate, lithium hydrogen oxalate, calcium oxalate, barium oxalate, magnesium oxalate, zinc oxalate, manganese oxalate, nickel oxalate, strontium oxalate, lead oxalate, ammonium malonate, ammonium hydrogen malonate, sodium malonate, sodium hydrogen malonate, potassium malonate, potassium hydrogen malonate, lithium malonate, lithium hydrogen malonate, calcium malonate, barium malonate, magnesium malonate, zinc malonate, manganese malonate, nickel malonate, strontium malonate, lead malonate, ammonium succinate, sodium succinate, potassium succinate, lithium succinate, calcium succinate, barium succinate, magnesium succinate, zinc succinate, manganese succinate, nickel succinate, strontium succinate, lead succinate, ammonium glutarate, sodium glutarate, potassium glutarate, lithium glutarate, calcium glutarate, barium glutarate, magnesium glutarate, zinc glutarate, manganese glutarate, nickel glutarate, strontium glutarate, lead glutarate, ammonium phthalate, sodium phthalate, potassium phthalate, lithium phthalate, calcium phthalate, barium phthalate, magnesium phthalate, zinc phthalate, nickel phthalate, strontium phthalate, and lead phthalate, etc. Among them, zinc acetate, magnesium acetate, aluminum acetylacetonate, aluminum chloride, gallium acetylacetonate, gallium chloride, zinc acetylacetonate, zinc chloride, diethylzinc, and ZnMgO are preferred as the substance to be contained in the electron transport layer 60.

The reactants of the aforementioned substances refer to intermediate products that have been partially or wholly hydrolyzed or that have been partially condensed. Specifically, for example, a reactant, which is formed by coating a solution containing the aforementioned substance onto a substrate and then by heating the solution at a temperature (t₁) of 100°C ≤ t₁ ≤ 150°C, is preferred; or when X in the chemical formula (1) is a carboxylate group or an acetonate group, a reactant, which is contained in an electron transport layer having a carboxyl group absorption coefficient (α) of 0.5 × 10⁵ cm⁻¹ ≤ α ≤ 2.5 × 10⁵ cm⁻¹, is preferred.

The conduction band bottom energy of the electron transport layer 40 is preferably 4.4 eV or less, more preferably 4.2 eV or less, and still more preferably 4.0 eV or less. The conduction band bottom energy of the electron transport layer 60 can be calculated by deducting a bandgap determined by ultraviolet-visible absorption spectrum from an ionization potential determined by ultraviolet photoelectron spectroscopy.

The electron transport layer 40 of the present embodiment can be formed by applying a solution containing a material represented by the aforementioned chemical formula (1) and then by subjecting to a heat treatment. When a solution containing a material represented by the following chemical formula (1-1) is used, it is particularly desirable to conduct the heat treatment at a temperature (t₁) of 100°C ≤ t₁ ≤ 150°C. If the heat treatment temperature is lower than 100°C, the film does not function as an electron transport layer, and hence the photoelectric conversion performance is drastically decreased, or photoelectric conversion is not performed at all. On the other hand, if the heat treatment temperature is 150°C or higher, the conduction band bottom energy becomes too high, and hence the photoelectric conversion performance is decreased.

Zn(X)₂ (1-1)

wherein X is selected from a halogen, carboxylate group, alkoxy group, alkyl group, and acetonate group represented by the following formula: 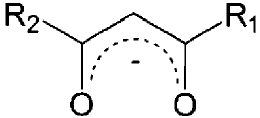 wherein R₁ and R₂ are selected from hydrogen, a C₁₋₂₀ linear or branched alkyl group, and C₁₋₂₀ linear or branched alkoxy group, and R₁ and R₂ may or may not be the same as each other. Examples of the alkyl group include, for example, a methyl group, ethyl group, and propyl group, etc.; and examples of the alkoxy group include, for example, a methoxy group and ethoxy group, etc.

When a solution containing a material represented by the following chemical formula (1-2), in addition to the aforementioned chemical formula (1-1), is used, it is desirable to conduct the heat treatment at a temperature of 300°C or higher. If the heat treatment temperature is lower than 300°C, the film does not function as an electron transport layer, and hence the photoelectric conversion performance is drastically decreased, or photoelectric conversion is not performed at all.

Mg(X)₂ (1-2)

wherein X is selected from a halogen, carboxylate group, alkoxy group, alkyl group, and acetonate group represented by the following formula: 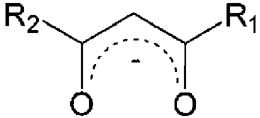 wherein R₁ and R₂ are selected from hydrogen, a C₁₋₂₀ linear or branched alkyl group, and C₁₋₂₀ linear or branched alkoxy group, and R₁ and R₂ may or may not be the same as each other. Examples of the alkyl group include, for example, a methyl group, ethyl group, and propyl group, etc.; and examples of the alkoxy group include, for example, a methoxy group and ethoxy group, etc.

A solution to be used in forming the electron transport layer 40 can be manufactured by dissolving the material represented by the chemical formula (1) in a predetermined solvent. The solvent is not particularly limited, as far as the material represented by the chemical formula (1) can be dissolved therein, but examples thereof include alcohol-based solvents, such as methanol, ethanol, isopropanol, 1-propanol, 2-methoxyethanol, and 2-ethoxyethanol, etc., and mixtures thereof.

The concentration of the material represented by the chemical formula (1) in the solution is not particularly limited, but it is 1 mg to 1 g/ml, preferably 5 mg to 500 mg/ml, and more preferably 10 mg to 100 mg /ml.

Examples of the preferred material for forming the electron transport layer include zinc acetate, magnesium acetate, aluminum acetylacetonate, aluminum chloride, gallium acetylacetonate, gallium chloride, zinc acetylacetonate, zinc chloride, and diethylzinc, and among them, zinc acetate is most preferred.

A method of forming the electron transport layer 40 is not particularly limited, but various methods, such as a spin coating method, die coating method, gravure printing method, ink jet method, spray method, and screen printing method, can be adopted.

The conduction band bottom energy adjustment layer 50 is interposed between the photoelectric conversion layer 60 and the electron transport layer 40. The conduction band bottom energy adjustment layer 50 functions to reduce the conduction band bottom energy of the electron extraction layer 90 to energy lower than that of the electron transport layer 40. An amount of a reduction in the conduction band bottom energy of the electron extraction layer 90 by the conduction band bottom energy adjustment layer 50 is not particularly limited, but it is preferably 0.8 eV or more, and more preferably 1.1 eV or more. The conduction band bottom energy of each of the electron extraction layer 90 and the electron transport layer 40 can be measured by using photoelectron spectroscopy. The thickness of the conduction band bottom energy adjustment layer 50 has only to be greater than or equal to a monolayer, and is preferably 0.5 to 50 nm.

A compound containing an alkali metal or an alkaline earth metal is preferred as a material to form the conduction band bottom energy adjustment layer 50, and examples of the compound include a sodium compound, potassium compound, rubidium compound, cesium compound, magnesium compound, potassium compound, strontium compound, and barium compound, etc. Specifically, carbonates, nitrates, acetates, sulfates, halides, and acetonato complexes, etc., all of which contain the aforementioned metal, can be cited. More specific examples of the compound include sodium carbonate, potassium carbonate, rubidium carbonate, cesium carbonate, magnesium carbonate, potassium carbonate, strontium carbonate, barium carbonate, sodium nitrate, potassium nitrate, rubidium nitrate, cesium nitrate, magnesium nitrate, potassium nitrate, strontium nitrate, barium nitrate, sodium acetate, potassium acetate, rubidium acetate, cesium acetate, magnesium acetate, potassium acetate, strontium acetate, barium acetate, sodium sulfate, potassium sulfate, rubidium sulfate, cesium sulfate, magnesium sulfate, potassium sulfate, strontium sulfate, barium sulfate, sodium fluoride, potassium fluoride, rubidium fluoride, cesium fluoride, magnesium fluoride, potassium fluoride, strontium fluoride, barium fluoride, sodium chloride, potassium chloride, rubidium chloride, cesium chloride, magnesium chloride, potassium chloride, strontium chloride, barium chloride, sodium bromide, potassium bromide, rubidium bromide, cesium bromide, magnesium bromide, potassium bromide, strontium bromide, barium bromide, sodium iodide, potassium iodide, rubidium iodide, cesium iodide, magnesium iodide, potassium iodide, strontium iodide, barium iodide, sodium acetylacetonate, potassium acetylacetonate, rubidium acetylacetonate, cesium acetylacetonate, magnesium acetylacetonate, potassium acetylacetonate, strontium acetylacetonate, and barium acetylacetonate, etc.

A method of forming the conduction band bottom energy adjustment layer 50 is not particularly limited, but various methods, such as a spin coating method, die coating method, gravure printing method, ink jet method, spray method, screen printing method, and vacuum deposition method, can be adopted.

As described above, the conduction band bottom energy adjustment layer 50 functions to reduce the conduction band bottom energy of the electron extraction layer 90 to energy lower than that of the electron transport layer 40, but in some cases functions to transport electrons from the photoelectric conversion layer 60 to the first electrode 30. In this case, the conduction band bottom energy adjustment layer 50 can be regarded as part of the electron transport layer 40.

The photoelectric conversion layer 60 of the present embodiment is a bulk heterojunction layer, and is formed by mixing, at a nano level, a p-type organic semiconductor having an electron donating property and an n-type organic semiconductor having an electronic accepting property. Examples of the p-type organic semiconductor include electron donating molecules, such as charge transfer agents and charge transfer complexes, the charge transfer agents including: polythiophenes, such as poly (3-hexylthiophene), and oligomers thereof; organic pigment molecules, such as polypyrrole, polyaniline, polyfuran, polypyridine, polycarbazole, phthalocyanine, porphyrin, and perylene, and derivatives and transition metal complexes thereof; triphenylamine compounds; and hydrazine compounds, and the charge transfer complexes including tetra rear full Burren (TTF), etc.

Examples of the n-type organic semiconductor include: fullerene and fullerene derivatives, such as [60]PCBM (phenyl C61 butyric acid methyl ester), bis[60]PCBM, ICMA (Indene-C60 monoadduct), ICBA (Indene-C60 bisadduct), and [70]PCBM (phenyl C71 butyric acid methyl ester); carbon materials, such as carbon nanotube and chemically-modified carbon nanotube; metal complexes having, as a ligand, (1) a condensed ring aromatic compound (naphthalene derivative, anthracene derivative, phenanthrene derivative, tetracene derivative, pyrene derivative, perylene derivative, or fluoranthene derivative), (2) a 5- to 7-membered heterocycle compound containing a nitrogen atom, oxygen atom, and sulfur atom (e.g., pyridine, pyrazine, pyrimidine, pyridazine, triazine, quinoline, quinoxaline, quinazoline, phthalazine, cinnoline, isoquinoline, pteridine, acridine, phenazine, phenanthroline, tetrazole, pyrazole, imidazole, thiazole, oxazol, indazole, benzimidazole. benzotriazol, benzoxazole, benzothiazole, carbazole, purine, triazolopyridazine, triazolopyrimidine, tetrazaindene, oxadiazole, imidazopyridine, pyrralidine, pyrrolopyridine, thiadiazolopyridine, dibenzazepine, tribenzazepine, or the like), (3) a polyarylene compound, (4) a fluorene compound, (5) a cyclopentadiene compound, (6) a silyl compound, or (7) a nitrogen-containing heterocyclic compound; and the like.

The n-type organic semiconductor is preferably fullerene or a fullerene derivative. Herein, the fullerene represents C60, C70, C76, C78, C80, C82, C84, C90, C96, C240, C540, mixed fullerene, or fullerene nanotube, and the fullerene derivative represents a compound in which a substituent group is added to the fullerene.

In the present description, when a specific portion is referred to as a "group", it means that the portion itself may or may not be substituted by one or more (up to the possible largest number) substituent groups. For example, an "alkyl group" means a substituted or unsubstituted alkyl group. The substituent group that can be used in a compound in the present description may be any substituent group.

When such a substituent group is represented by W, the substituent group represented by W is not particularly limited, and may be any substituent group. Examples of the substituent group represented by W include, for example: halogen atoms; alkyl groups (including a cycloalkyl group, bicycloalkyl group, and tricycloalkyl group); alkenyl groups (including a cycloalkenyl group and bicycloalkenyl group); alkynyl groups; aryl groups; heterocyclic groups (also referred to as hetero ring groups); cyano groups; hydroxyl groups; nitro groups; carboxyl groups; alkoxy groups; aryloxy groups; silyloxy groups; heterocyclic oxy groups; acyloxy groups; carbamoyloxy groups; alkoxy carbonyloxy groups; aryloxy carbonyloxy groups; amino groups (including an anilino group); ammonio groups; acylamino groups; aminocarbonyl amino groups; alkoxycarbonylamino groups; aryloxycarbonylamino groups; sulfamoylamino groups; alkyl or aryl sulfonylamino groups; mercapt groups; alkylthio groups; arylthio groups; heterocyclic thio groups; sulfamoyl groups; sulfo groups; alkyl or aryl sulfinyl groups; alkyl or aryl sulfonyl groups; acyl groups; aryloxy carbonyl groups; alkoxycarbonyl groups; carbamoyl groups; aryl or heterocyclic azo groups; imido groups; phosphino groups; phosphinyl groups; phosphinyloxy groups; phosphinyl amino groups; phosphono groups; silyl groups; hydrazino groups; ureido groups; boronic acid groups (-B(OH)₂); phosphato groups (-OPO(OH)₂); sulfato groups (-OSO₃H); and other publicly-known substituent groups.

In more detail, W represents: halogen atoms (e.g., a fluorine atom, chlorine atom, bromine atom, and iodine atom); and alkyl groups [linear, branched, and cyclic substituted or unsubstituted alkyl groups]. Examples of them include: alkyl groups (preferably C₁₋₃₀ alkyl groups, such as, for example, methyl, ethyl, n-propyl, isopropyl, t-butyl, n-octyl, eicosyl, 2-chloroethyl, 2-cyanoethyl, 2-ethylhexyl); cycloalkyl groups (preferably C₃₋₃₀ substituted or unsubstituted cycloalkyl groups, such as, for example, a cyclohexyl group, cyclopentyl group, and 4-n-dodecylcyclohexyl group); bicycloalkyl groups (preferably C₅₋₃₀ substituted or unsubstituted bicycloalkyl groups, i.e., monovalent groups obtained by removing one hydrogen atom from C₅₋₃₀ bicycloalkanes, such as, for example, a bicyclo[1,2,2]heptane-2-yl group and bicyclo[2,2,2]octane-3-yl group); and tricyclo structures having more cyclic structures, etc. The alkyl groups in the substituent groups described below (e.g., an alkyl group in an alkylthio group) represent alkyl groups of such a concept, and are meant to further include alkenyl groups and alkynyl groups.]; and alkenyl groups [linear, branched, and cyclic substituted or unsubstituted alkenyl groups]. Examples of them include: alkenyl groups (preferably C₂₋₃₀ substituted or unsubstituted alkenyl groups, such as, for example, a vinyl group, allyl group, prenyl group, geranyl group, and oleyl group); cycloalkenyl groups (preferably C₃₋₃₀ substituted or unsubstituted cycloalkenyl groups, i.e., monovalent groups obtained by removing one hydrogen atom from C₃₋₃₀ cycloalkenes, such as, for example, a 2-cyclopentene-1-yl group and 2-cyclohexene-1-yl group); bicycloalkenyl groups (substituted or unsubstituted bicycloalkenyl groups, preferably C₅₋₃₀ substituted or unsubstituted bicycloalkenyl groups, i.e., monovalent groups obtained by removing one hydrogen atom from bicycloalkene groups having one double bond, such as, for example, a bicyclo[2,2,1]hept-2-ene-1-yl group and bicyclo[2,2,2]oct-2-ene-4-yl group];
alkynyl groups (preferably C₂₋₃₀ substituted or unsubstituted alkynyl groups, such as, for example, an ethynyl group, propargyl group, and trimethylsilyl ethynyl group); aryl groups (preferably C₆₋₃₀ substituted or unsubstituted aryl groups, such as, for example, a phenyl group, p-tolyl group, naphthyl group, m-chlorophenyl group, and o-hexadecanoyl aminophenyl group); heterocyclic groups (preferably substituted or unsubstituted monovalent groups having 5 or 6 members that are obtained by removing one hydrogen atom from aromatic or non-aromatic heterocyclic compounds, and more preferably C₃₋₃₀ aromatic heterocyclic groups having 5 or 6 members, such as, for example, a 2-furyl group, 2-thienyl group, 2-pyrimidinyl group, and 2-benzothiazolyl group. Further, cationic heterocyclic groups, such as a 1-methyl-2-pyridinio group and 1-methyl-2-quinolinio group may be included.); cyano groups; hydroxyl groups; nitro groups; carboxyl groups; alkoxy groups (preferably C₁₋₃₀ substituted or unsubstituted alkoxy groups, such as, for example, a methoxy group, ethoxy group, isopropoxy group, t-butoxy group, n-octyloxy group, and 2-methoxyethoxy group); aryloxy groups (preferably C₆₋₃₀ substituted or unsubstituted aryloxy groups, such as, for example, a phenoxy group, 2-methylphenoxy group, 4-t-butylphenoxy group, 3-nitrophenoxy group, and 2-tetradecanoyl aminophenoxy group); silyloxy groups (preferably C₃₋₂₀ silyloxy groups, such as, for example, a trimethylsilyloxy group and t-butyldimethylsilyloxy group); heterocyclic oxy groups (preferably C₂₋₃₀ substituted or unsubstituted heterocyclic oxy group groups, such as a 1-phenyltetrazole-5-oxy group and 2-tetrahydropyranyloxy group); acyloxy groups (preferably a formyloxy group, C₂₋₃₀ substituted or unsubstituted alkylcarbonyloxy groups, and C₆₋₃₀ substituted or unsubstituted aryl carbonyloxy groups, such as, for example, a formyloxy group, acetyloxy group, pivaloyloxy group, stearoyloxy group, benzoyloxy group, and p-methoxyphenyl carbonyloxy group); carbamoyloxy groups (preferably C₁₋₃₀ substituted or unsubstituted carbamoyloxy groups, such as, for example, an N,N-dimethylcarbamoyloxy group, N,N-diethylcarbamoyloxy group, morpholino carbonyloxy group, N,N-di-n-octyl aminocarbonyl oxy group, and N-n-octyl carbamoyloxy group); alkoxy carbonyloxy groups (preferably C₂₋₃₀ substituted or unsubstituted alkoxy carbonyloxy groups, such as, for example, a methoxycarbonyloxy group, ethoxycarbonyloxy group, t-buthoxycarbonyloxy group, and n-octyl carbonyloxy group); aryloxy carbonyloxy groups (preferably C₇₋₃₀ substituted or unsubstituted aryloxy carbonyloxy groups, such as, for example, a phenoxy carbonyloxy group, p-methoxy phenoxy carbonyloxy group, and p-n-hexadecyloxy phenoxy carbonyloxy group); amino groups (preferably an amino group, C₁₋₃₀ substituted or unsubstituted alkylamino groups, and C₆₋₃₀ substituted or unsubstituted anilino groups, such as, for example, an amino group, methylamino group, dimethylamino group, anilino group, N-methyl-anilino group, and diphenylamino group); ammonio groups (preferably an ammonio group, C₁₋₃₀ substituted or unsubstituted ammonio groups in which an alkyl group, aryl group, or heterocyclic group is substituted, such as, for example, a trimethylammonio group, triethylammonio group, and diphenyl methylammonio group); acylamino groups (preferably a formylamino group, C₁₋₃₀ substituted or unsubstituted alkyl carbonylamino groups, and C₆₋₃₀ substituted or unsubstituted aryl carbonylamino groups, such as, for example, a formylamino group, acetylamino group, pivaloylamino group, lauroylamino group, benzoylamino group, and 3,4,5-tri-n-octyloxy phenyl carbonylamino group); aminocarbonyl amino groups (preferably C₁₋₃₀ substituted or unsubstituted aminocarbonyl amino groups, such as, for example, a carbamoyl amino group, N,N-dimethylaminocarbonylamino group, N,N-diethylaminocarbonylamino group, and morpholinocarbonylamino group); alkoxycarbonylamino groups (preferably C₂₋₃₀ substituted or unsubstituted alkoxycarbonylamino groups, such as, for example, a methoxycarbonylamino group, ethoxycarbonylamino group, t-butoxycarbonylamino group, n-octadecyloxycarbonylamino group, and N-methyl-methoxycarbonylamino group); aryloxy carbonylamino groups (preferably C₇₋₃₀ substituted or unsubstituted aryloxy carbonylamino groups, such as, for example, a phenoxycarbonylamino group, p-chlorophenoxycarbonylamino group, and m-n-octyloxyphenoxycarbonylamino group); sulfamoylamino groups(preferably C₀₋₃₀ substituted or unsubstituted sulfamoylamino groups, such as, for example, a sulfamoylamino group, N,N-dimethylaminosulfonylamino group, and N-n-octylaminosulfonylamino group); alkyl and arylsulfonyl amino groups (preferably C₁₋₃₀ substituted or unsubstituted alkylsulfonyl amino groups and C₆₋₃₀ substituted or unsubstituted arylsulfonyl amino groups, such as, for example, a methylsulfonyl amino group, butylsulfonylamino group, phenylsulfonyl amino group, 2,3,5-trichlorophenylsulfonylamino group, and p-methylphenylsulfonylamino group); mercapt groups; alkylthio groups (preferably C₁₋₃₀ substituted or unsubstituted alkylthio groups, such as, for example, a methylthio group, ethylthio group, and n-hexadecylthio group); arylthio groups (preferably C₆₋₃₀ substituted or unsubstituted arylthio groups, such as, for example, a phenylthio group, p-chlorophenylthio group, and m-methoxyphenylthio group); heterocyclic thio groups (preferably C₂₋₃₀ substituted or unsubstituted heterocyclic thio groups, such as, for example, a 2-benzothiazolylthio group and 1-phenyltetrazole-5-ylthio group); sulfamoyl groups (preferably C₀₋₃₀ substituted or unsubstituted sulfamoyl groups, such as, for example, an N-ethylsulfamoyl group, N-(3-dodecyloxy propyl)sulfamoyl group, N,N-dimethyl sulfamoyl group, N-acetylsulfamoyl group, N-benzoylsulfamoyl group, and N-(N'-phenylcarbamoyl)sulfamoyl group); sulfo groups; alkyl and aryl sulfinyl groups (preferably C₁₋₃₀ substituted or unsubstituted alkyl sulfinyl groups and C₆₋₃₀ substituted or unsubstituted aryl sulfinyl groups, such as, for example, a methyl sulfinyl group, ethyl sulfinyl group, phenyl sulfinyl group, and p-methylphenylsulfinyl group); alkyl and aryl sulfonyl groups (preferably C₁₋₃₀ substituted or unsubstituted alkyl sulfonyl groups and C₆₋₃₀ substituted or unsubstituted aryl sulfonyl groups, such as, for example, a methyl sulfonyl group, ethyl sulfonyl group, phenyl sulfonyl group, and p-methylphenyl sulfonyl group); acyl groups (preferably a formyl group, C₂₋₃₀ substituted or unsubstituted alkyl carbonyl groups, C₇₋₃₀ substituted or unsubstituted aryl carbonyl groups, and C₄₋₃₀ substituted or unsubstituted heterocyclic carbonyl groups bonded to a carbonyl group by a carbon atom, such as, for example, an acetyl group, pivaloyl group, 2-chloroacetyl group, stearoyl group, benzoyl group, p-n-octyloxyphenylcarbonyl group, 2-pyridyl carbonyl group and 2-furylcarbonyl group); aryloxy carbonyl groups (preferably C₇₋₃₀ substituted or unsubstituted aryloxy carbonyl groups, such as, for example, a phenoxy carbonyl group, o-chlorophenoxycarbonyl group, m-nitrophenoxycarbonyl group, and p-t-butylphenoxycarbonyl group); alkoxycarbonyl groups (preferably C₂₋₃₀ substituted or unsubstituted alkoxycarbonyl groups, such as, for example, a methoxycarbonyl group, ethoxycarbonyl group, t-butoxycarbonyl group, and n-octadecyloxycarbonyl group); carbamoyl groups (preferably C₁₋₃₀ substituted or unsubstituted carbamoyl groups, such as, for example, a carbamoyl group, N-methylcarbamoyl group, N,N-dimethylcarbamoyl group, N,N-di-n-octylcarbamoyl group, and N-(methylsulfonyl)carbamoyl group); aryl and heterocyclic azo groups (preferably C₆₋₃₀ substituted or unsubstituted aryl azo groups, C₃₋₃₀ substituted or unsubstituted heterocyclic azo groups, such as, for example, a phenyl azo group, p-chlorophenylazo group, and 5-ethylthio-1,3,4-thiadiazole-2-ylazo group); imido groups (preferably an N-succinimide group and N-phthalimide group); phosphino groups (preferably C₂₋₃₀ substituted or unsubstituted phosphino groups, such as, for example, a dimethylphosphino group, diphenylphosphino group, and methylphenoxyphosphino group); phosphinyl groups (preferably C₂₋₃₀ substituted or unsubstituted phosphinyl groups, such as, for example, a phosphinyl group, dioctyloxyphosphinyl group, and diethoxyphosphinyl group); phosphinyloxy groups (preferably C₂₋₃₀ substituted or unsubstituted phosphinyloxy groups, such as, for example, a diphenoxyphosphinyloxy group and dioctyloxyphosphinyloxy group); phosphinyl amino groups (preferably C₂₋₃₀ substituted or unsubstituted phosphinyl amino groups, such as, for example, a dimethoxyphosphinylamino group and dimethylaminophosphinylamino group); phospho groups; silyl groups (preferably C₃₋₃₀ substituted or unsubstituted silyl groups, such as, for example, a trimethylsilyl group, t-butyldimethylsilyl group, and phenyl dimethylsilyl group); hydrazino groups (preferably C₀₋₃₀ substituted or unsubstituted hydrazino grous, such as, for example, a trimethylhydrazino group); or ureide groups (preferably C₀₋₃₀ substituted or unsubstituted ureide groups, such as, for example, an N,N-dimethylureide group).

Two Ws can also cooperate to form rings (aromatic or non-aromatic hydrocarbon rings or hetero rings that can be further combined together to form a polycyclic condensed ring. Examples of the rings include, for example, a benzene ring, naphthalene ring, anthracene ring, phenanthrene ring, fluorene ring, triphenylene ring, naphthacene ring, biphenyl ring, pyrrole ring, furan ring, thiophene ring, imidazole ring, oxazole ring, thiazole ring, pyridine ring, pyrazine ring, pyrimidine ring, pyridazine ring, indolizine ring, indole ring, benzofuran ring, benzothiophene ring, isobenzofuran ring, benzimidazole ring, imidazopyridine ring, quinolizine ring, quinoline ring, phthalazine ring, naphthyridine ring, quinoxaline ring, quinoxazolin ring, isoquinoline ring, carbazole ring, phenanthridine ring, acridine ring, phenanthroline ring, thianthrene ring, chromene ring, xanthene ring, phenoxathiin ring, phenothiazine ring, and phenazine ring).

Of the aforementioned substituent groups represented by W, a substituent group having a hydrogen atom may be further substituted by the aforementioned groups after the hydrogen atom is removed. Examples of such a substituent group include -CONHSO₂-groups (a sulfonylcarbamoyl group and carbonylsulfamoyl group), -CONHCO-groups (a carbonylcarbamoyl group), and - SO₂NHSO₂-groups (a sulfonyl sulfamoyl group). More specific examples thereof include alkylcarbonylaminosulfonyl groups (e.g., an acetylaminosulfonyl group), arylcarbonylaminosulfonyl groups (e.g., a benzoylaminosulfonyl group), alkylsulfonylaminocarbonyl groups (e.g., a methylsulfonylaminocarbonyl group), and arylsulfonylaminocarbonyl groups (e.g., a p-methylphenylsulfonylaminocarbonyl group).

As a fullerene derivative to be used preferably, a compound represented by the following general formula (2) is cited.

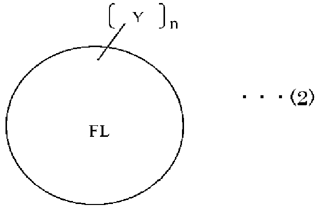

In the general formula (2), FL with a circle frame represents a fullerene C60, C70, or C84. Y represents a substituent group. As the substituent group, the aforementioned W can be used. Preferred examples of the substituent group include an alkyl group, an aryl group, and a heterocyclic group, and preferred specific examples thereof include those described with respect to W. More preferred examples of the alkyl group include C₁₋₁₂ alkyl groups; and preferred examples of the aryl group and the heterocyclic group include a benzene ring, naphthalene ring, anthracene ring, phenanthrene ring, fluorene ring, triphenylene ring, naphthacene ring, biphenyl ring, pyrrole ring, furan ring, thiophene ring, imidazole ring, oxazole ring, thiazole ring, pyridine ring, pyrazine ring, pyrimidine ring, pyridazine ring, indolizine ring, indole ring, benzofuran ring, benzothiophene ring, isobenzofuran ring, benzimidazole ring, imidazopyridine ring, quinolizine ring, quinoline ring, phthalazine ring, naphthyridine ring, quinoxaline ring, quinoxazolin ring, isoquinoline ring, carbazole ring, phenanthridine ring, acridine ring, phenanthroline ring, thianthrene ring, chromene ring, xanthene ring, phenoxathiin ring, phenothiazine ring, and phenazine ring. More preferred examples thereof include a benzene ring, naphthalene ring, anthracene ring, phenanthrene ring, pyridine ring, imidazole ring, oxazole ring, and thiazole ring. Particularly preferred examples thereof include a benzene ring, naphthalene ring, and pyridine ring. These substituent groups may further have substituent groups that may be bonded as much as possible to form rings. Herein, when n is 2 or more, multiple Ys may or may not be the same as each other, and multiple Xs may be bonded as much as possible to form rings. n represents an integer of 1 to 60, but is preferably an integer of 1 to 10.

Hereinafter, specific examples of a fullerene derivative to be preferably used in the present embodiment will be described, but the embodiment should not be limited thereto.

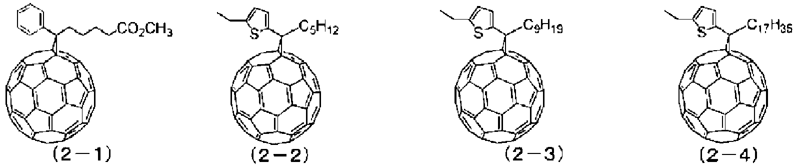

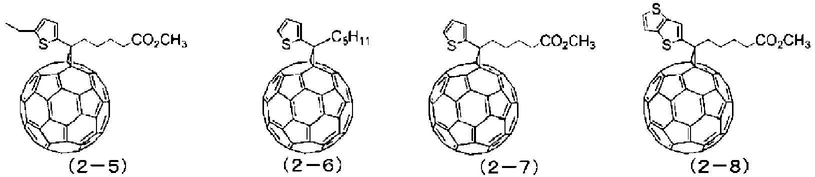

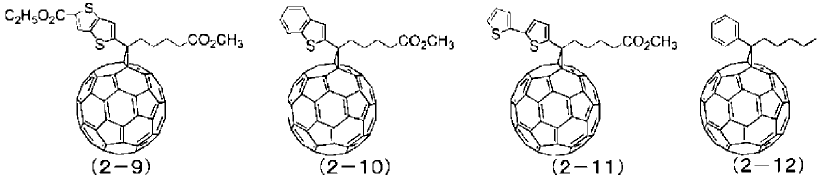

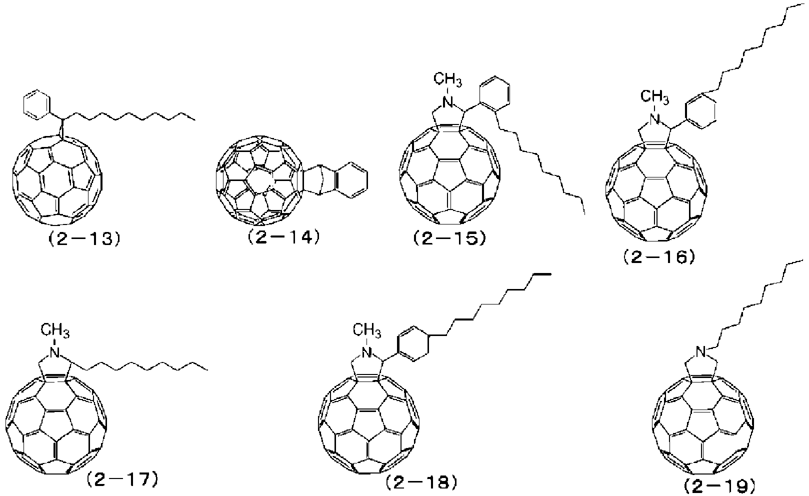

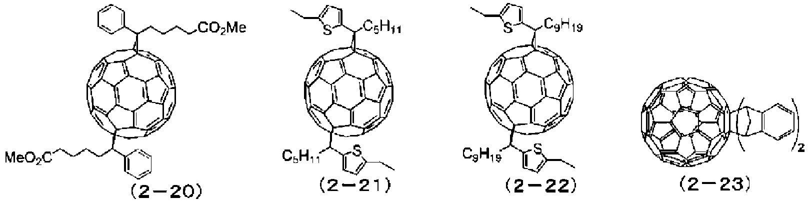

Among the specific examples, (2-1), (2-2), (2-3), (2-4), (2-12), (2-13), (2-20), (2-21), (2-22), and (2-23) are preferred, and (2-20), (2-21), (2-22), and (2-23) are more preferred.

Alternatively, as the fullerene and fullerene derivative to be used in the present embodiment, the compounds described in the following documents can also be used, the documents being: Quarterly Chemistry Survey, No. 43 (1999), edited by Chemical Society of Japan; Japanese Patent Application Publication No. 1998-167994; Japanese Patent Application Publication No. 1999-255508; Japanese Patent Application Publication No. 1999-255509; Japanese Patent Application Publication No. 2002-241323; and Japanese Patent Application Publication No. 2003-196881, etc. The fullerenes and fullerene compounds to be used in the embodiment can be produced, for example, by the methods described in the aforementioned documents, or by methods according to the methods described therein. Among these electron-accepting molecules, by using ICBA as the n-type organic semiconductor, the open circuit voltage (V_{oc}) can be improved.

When a fullerene derivative is used as the n-type organic semiconductor, it is preferable that the first reduction potential thereof is 1160 mV (vs Fc/Fc⁺) or more, more preferable that the potential is 1250 mV (vs Fc/Fc⁺) or more, and still more preferable that the potential is 1350 mV (vs Fc/Fc⁺) or more. Measurement of the first reduction potential of the n-type organic semiconductor can be carried out as follows, with reference to "Electochemical Methods: Fundamentals and Applications" written by A. J. Bard. An o-dichlorobenzene 0.1M solution of tetrabutylammonium perchlorate was manufactured, and 4 mg of ferrocene was added, as an internal reference substance, to per 50 mL of the solution to prepare a measuring solution. To 2 mL of this solution, 1.5 mg of a fullerene derivative was added, and an oxidation-reduction potential was measured at a sweep rate of 20 mV/s by using a potentiostat galvanostat (electrochemical analyzer: model 630A made by ALS). A first reduction potential was determined as the average of the first reduction peak and its oxidation peak, based on the oxidation/reduction potential (Fc/Fc⁺) of the ferrocene added as an internal reference substance.

The thickness of the photoelectric conversion layer 60 is not particularly limited, but it is 5 to 1000 nm, preferably 10 to 500 nm, more preferably 20 to 200 nm, and still more preferably 40 to 100 nm. There is a tendency that, as the thickness of the photoelectric conversion layer 60 is smaller, light resistance is more improved.

The hole transport layer 70 is provided in a region between the second electrode 80 and the photoelectric conversion layer 60. The hole transport layer 70 functions to facilitate the transport of holes from the photoelectric conversion layer 60 to the second electrode 80. The hole transport layer 70 can also function such that the transport of electrons from the photoelectric conversion layer 60 to the second electrode 80 is hardly caused. The hole transport layer 70 is formed of a material having a high hole mobility, such as a charge transfer agent, a charge transfer complex, or the like. Examples of the charge transfer agent include, for example: conductive polymers, such as PEDOT (polythiophene, poly(ethylenedioxy)thiophene)/PSS (poly(styrenesulfonate)), polypyrrole, polyaniline, polyfuran, polypyridine, and polycarbazole; inorganic compounds, such as MoO₃ and WO₃; organic semiconductor molecules, such as phthalocyanine and porphyrin, and derivatives and transition metal complexes thereof; triphenylamine compounds; and hydrazine compounds, etc. Examples of the charge transfer complex include, for example, tetra rear full Burren (TTF). The thickness of the hole transport layer is not particularly limited, but it is 10 to 100 nm, and preferably 20 to 60 nm.

The second electrode 80 of the present embodiment is a positive electrode (hole extraction electrode), and is electrically connected to the photoelectric conversion layer 60 via the hole transport layer 70 and on the side of the photoelectric conversion layer 60 opposite to its light-receiving surface. The material of the second electrode 80 is not particularly limited, as far as the material has conductivity, but a metal, such as gold, platinum, silver, copper, aluminum, magnesium, lithium, potassium, or the like; a carbon electrode; or the like can be used. The second electrode 80 can be formed by a publicly-known method, such as a vacuum deposition method, electron beam vacuum deposition method, sputtering method, method in which metal fine particles dispersed in a solvent are coated and the solvent is then volatilized and removed.

A means for blocking ultraviolet rays can be incorporated in the photoelectric conversion element 10. The means for blocking ultraviolet rays is not particularly limited, as far as the element can be blocked from ultraviolet rays, but examples of the means include an ultraviolet absorption layer, ultraviolet reflecting layer, and wavelength conversion layer for converting the wavelength of an ultraviolet ray into another wavelength, etc.

A position, at which the means for blocking ultraviolet rays is provided, is not particularly limited, as far as the element can be blocked from ultraviolet rays, but the means is provided in one of the following ways: a layer having the aforementioned function of blocking ultraviolet rays is provided on the surface on the light emission side of the substrate; a film having the function is attached to the surface; a substrate having the function is used as the substrate on the light emission side; a layer having the function is provided between the substrate on the light emission side and the transparent conducting film; when the element has a sub-straight structure (a structure laminated from the metal electrode side), a sealing material having the function is used; and the like.

A range of the wavelength of the ultraviolet ray to be blocked is not particularly limited, but it is 330 nm or less, preferably 350 nm or less, more preferably 370 nm or less, still more preferably 390 nm or less, and still more preferably 400 nm or less. The transmissivity of the ultraviolet ray is 10% or less, preferably 1% or less, and more preferably 0.1% or less.

When xenon lamp light, the ultraviolet ray intensity of which is adjusted to be equivalent to 1 Sun, is continuously emitted on the photoelectric conversion element 10 that is in a state where the light is not emitted (initial state) at ambient temperature of 40°C for 1000 hours, a decrease rate of the photoelectric conversion efficiency (a decrease amount of the photoelectric conversion efficiency after the continuous emission for 1000 hours/the photoelectric conversion efficiency in the initial state × 100) is preferably 10% or less. The adjustment of an ultraviolet ray intensity so as to be equivalent to 1 SUN can be achieved, for example, by adjusting, with an ultraviolet radiometer (MS-212A made by EKO Instruments), the illuminance of a xenon lamp such that an amount of ultraviolet rays having a wavelength of 315 to 400 nm becomes 45 W/m².

According to the photoelectric conversion element 10 of the present embodiment, the photoelectric conversion efficiency in the initial state where light is not emitted can not only be improved, but a decrease in the photoelectric conversion efficiency, possibly occurring in a state where light is continuously emitted for a long period of time, can be suppressed. As a result, the life of the photoelectric conversion element 10 can be extended.

Table 1 shows the conditions under which the photoelectric conversion elements of Examples 1 to 4 and Comparative Examples 1 to 12 were manufactured. Methods of manufacturing those photoelectric conversion elements will be described with reference to Table 1.

### (Example 1)

### <Formation of Negative Electrode>

An negative electrode (electron extraction electrode) was formed by cleaning a glass substrate (surface resistance value: 15 Ω/□) on which an ITO film had been formed by a sputtering method.

### <Formation of Electron Extraction Layer>

### (1) Formation of Electron Transport Layer

An electron transport layer was manufactured by a solution coating method. Specifically, zinc acetate dihydrate (made by Aldrich Co. LLC) was dissolved in 2-methoxyethanol such that the concentration thereof was 20 mg/ml, and further monoethanolamine was added (55 µl/ml) to prepare a solution. An electron transport layer was formed by spin-coating the solution on the aforementioned ITO for negative electrode at 2000 rpm (30 seconds) and then by subjecting the coated solution to a heat treatment on a hot plate at 120°C for 5 minutes (see Table 1). The thickness of the electron transport layer after the heat treatment was 30 nm.

### (2) Formation of Conduction Band Bottom Energy Adjustment Layer

Cesium carbonate (made by Aldrich Co. LLC) was dissolved in 2-ethoxyethanol such that the concentration thereof was 1.86 mg/ml to prepare a solution. A conduction band bottom energy adjustment layer was formed on the electron transport layer, which had been formed by the aforementioned method, by spin-coating the solution thereon at 5000 rpm (30 seconds) and then by subjecting the coated solution to a heat treatment on a hot plate at 150°C for 10 minutes. The thickness of the conduction band bottom energy adjustment layer after the heat treatment was 5 nm.

### <Formation of Photoelectric Conversion Layer>

P3HT and PCBM were mixed at a mass ratio of 1.0 : 1.0, and the mixture was dissolved in o-dichlorobenzene such that the total concentration thereof was 2.5% by mass. A photoelectric conversion layer was formed by spin-coating the solution over the substrate, over which the conduction band bottom energy adjustment layer had been formed, at 750 rpm (10 seconds).

### <Formation of Hole Transport Layer>

WO₃ was vacuum-deposited over the substrate, over which the photoelectric conversion layer had been manufactured, by a resistance heating method. The thickness of the WO₃ layer was 10 nm. The degree of vacuum during the vacuum deposition was set to be 10⁻⁶ Torr or less.

### <Formation of Positive Electrode>

Ag was vacuum-deposited over the substrate, over which up to the hole transport layer had been formed, by a resistance heating method. The thickness of the Ag layer was 100 nm. The degree of vacuum during the vacuum deposition was set to be 10⁻⁶ Torr or less.

### <Sealing Treatment>

The photoelectric conversion element (organic solar cell element) thus manufactured was attached to cover glass by using a thermosetting sealant to obtain a sealed element.

**[Table 1]**

| | PHOTOELECTRIC CONVERSION ELEMENT MANUFACTURING CONDITIONS | | | | | | | | | |
|---|---|---|---|---|---|---|---|---|---|---|
| | PHOTOELECTRIC CONVERSION LAYER (DONOR/ACCEPTOR) | ELECTRON EXTRACTION LAYER | | | | | | | | UV CUT PRESENCE/ ABSENCE |
| | | CONDUCTION BAND BOTTOM ENERGY ADJUSTMENT LAYER | | | | ELECTRON TRANSPORT LAYER | | | | |
| | | MATERIAL | THICKNESS [nm] | HEAT TREATMENT TEMPERATURE [°C] | HEAT TREATMENT TIME [min] | MATERIAL | THICKNESS [nm] | HEAT TREATMENT TEMPERATURE [°C] | HEAT TREATMENT TIME [min] | |
| EXAMPLE 1 | P3HT/ICBA | Cs₂CO₃ | 5 | 150 | 10 | Zn(OAc)₂ | 30 | 120 | 5 | ABSENCE |
| EXAMPLE 2 | P3HT/ICBA | Cs₂CO₃ | 5 | 150 | 10 | ZnO | 30 | 300 | 5 | ABSENCE |
| EXAMPLE 3 | P3HT/ICBA | Cs₂CO₃ | 5 | 150 | 10 | ZnMgO | 30 | 300 | 5 | ABSENCE |
| EXAMPLE 4 | P3HT/ICBA | Cs₂CO₃ | 5 | 150 | 10 | Zn(OAc)₂ | 30 | 120 | 5 | PRESENCE |
| EXAMPLE 5 | P3HT/ICBA | Cs₂CO₃ | 5 | 150 | 10 | ZnO | 30 | 300 | 5 | PRESENCE |
| EXAMPLE 6 | P3HT/ICBA | Cs₂CO₃ | 5 | 150 | 10 | ZnMgO | 30 | 300 | 5 | PRESENCE |
| COMPARATIVE EXAMPLE 1 | P3HT/ICBA | - | - | - | - | Zn(OAc)₂ | 30 | 120 | 5 | ABSENCE |
| COMPARATIVE EXAMPLE 2 | P3HT/ICBA | - | - | - | - | ZnO | 30 | 300 | 5 | ABSENCE |
| COMPARATIVE EXAMPLE 3 | P3HT/ICBA | - | - | - | - | ZnMgO | 30 | 300 | 5 | ABSENCE |
| COMPARATIVE EXAMPLE 4 | P3HT/ICBA | - | - | - | - | Cs₂CO₃ | 5 | 150 | 10 | ABSENCE |
| COMPARATIVE EXAMPLE 5 | P3HT/ICBA | Zn(OAc)₂ | 30 | 120 | 5 | ZnO | 30 | 300 | 5 | ABSENCE |
| COMPARATIVE EXAMPLE 6 | P3HT/ICBA | ZnMgO | 30 | 300 | 5 | ZnO | 30 | 300 | 5 | ABSENCE |
| COMPARATIVE EXAMPLE 7 | P3HT/ICBA | - | - | - | - | Zn(OAc)₂ | 30 | 120 | 5 | PRESENCE |
| COMPARATIVE EXAMPLE 8 | P3HT/ICBA | - | - | - | - | ZnO | 30 | 300 | 5 | PRESENCE |
| COMPARATIVE EXAMPLE 9 | P3HT/ICBA | - | - | - | - | ZnMgO | 30 | 300 | 5 | PRESENCE |
| COMPARATIVE EXAMPLE 10 | P3HT/ICBA | - | - | - | - | Cs₂CO₃ | 5 | 150 | 10 | PRESENCE |
| COMPARATIVE EXAMPLE 11 | P3HT/ICBA | Zn(OAc)₂ | 30 | 120 | 5 | ZnO | 30 | 300 | 5 | PRESENCE |
| COMPARATIVE EXAMPLE 12 | P3HT/ICBA | ZnMgO | 30 | 300 | 5 | ZnO | 30 | 300 | 5 | PRESENCE |

### (Examples 2, 3)

The method of manufacturing the photoelectric conversion element of Example 2 is the same as that in Example 1, except that the heat treatment temperature, occurring when the electron transport layer was formed, was 300°C. The method of manufacturing the photoelectric conversion element of Example 3 is the same as that in Example 1, except that a ZnMgO layer, as an electron transport layer, was formed as follows.

### <Formation of Electron Transport Layer>

Zinc acetate dihydrate (made by Aldrich Co. LLC) and magnesium acetate tetrahydrate (made by Aldrich Co. LLC) were respectively dissolved in 2-methoxyethanol such that their concentrations became 15 mg/ml and 5mg/ml, and further monoethanolamine was added (5.5 µl/ml) followed by stirring for 2 hours or longer. An electron transport layer was formed by spin-coating the solution on the aforementioned ITO for negative electrode at 2000 rpm (30 seconds) and then by subjecting the coated solution to a heat treatment on a hot plate at 300°C for 5 minutes. The thickness of the electron transport layer after the heat treatment was 30 nm.

### (Examples 4 to 6)

The methods of manufacturing the photoelectric conversion elements of Examples 4 to 6 were respectively the same as those in Examples 1 to 3, except that a UV-blocking film (KU-1000100 made by KING WORKS Co., Ltd., transmittance of light having a wavelength of 370 nm or less is 1% or less) was attached onto the glass substrate of the negative electrode.

### (Comparative Examples 1 to 4)

The methods of manufacturing the photoelectric conversion elements of Comparative Examples 1 to 3 were respectively the same as those in Examples 1 to 3, except that a conduction band bottom energy adjustment layer was not formed. The method of manufacturing the photoelectric conversion element of Comparative Example 4 was the same as that in Example 1, except that a conduction band bottom energy adjustment layer was not formed and a cesium carbonate layer, as an electron transport layer, was formed as follows.

### <Formation of Electron Transport Layer>

Cesium carbonate (made by Aldrich Co. LLC) was dissolved in 2-ethoxyethanol such that the concentration thereof was 1.86 mg/ml to prepare a solution. An electron transport layer was formed by spin-coating the solution on the aforementioned ITO for negative electrode at 5000 rpm (30 seconds) and then by subjecting the coated solution to a heat treatment on a hot plate at 150°C for 10 minutes. The thickness of the electron transport layer after the heat treatment was 5 nm.

### (Comparative Example 5)

The method of manufacturing the photoelectric conversion element of Comparative Example 5 was the same as that in Example 2, except that a zinc acetate layer, as a conduction band bottom energy adjustment layer, was formed as follows.

### <Formation of Conduction Band Bottom Energy Adjustment Layer>

Zinc acetate dihydrate (made by Aldrich Co. LLC) was dissolved in 2-methoxyethanol such that the concentration thereof was 20 mg/ml, and further monoethanolamine was added (55 µl/ml) to prepare a solution. A conduction band bottom energy adjustment layer was formed on the electron transport layer, which had been formed by the aforementioned method, by spin-coating the solution thereon at 2000 rpm (30 seconds) and then by subjecting the coated solution to a heat treatment on a hot plate at 120°C for 5 minutes. The thickness of the conduction band bottom energy adjustment layer after the heat treatment was 30 nm.

### (Comparative Example 6)

The method of manufacturing the photoelectric conversion element of Comparative Example 6 was the same as that in Example 2, except that a ZnMgO layer, as a conduction band bottom energy adjustment layer, was formed as follows.

### <Formation of Conduction Band Bottom Energy Adjustment Layer>

Zinc acetate dihydrate (made by Aldrich Co. LLC) and magnesium acetate tetrahydrate (made by Aldrich Co. LLC) were respectively dissolved in 2-methoxyethanol such that their concentrations became 15 mg/ml and 5mg/ml, and further monoethanolamine was added (5.5 µl/ml) followed by stirring for 2 hours or longer. A conduction band bottom energy adjustment layer was formed on the electron transport layer, which had been formed by the aforementioned method, by spin-coating the solution thereon at 2000 rpm (30 seconds) and then by subjecting the coated solution to a heat treatment on a hot plate at 300°C for 5 minutes. The thickness of the conduction band bottom energy adjustment layer after the heat treatment was 30 nm.

### (Comparative Examples 7 to 12)

The methods of manufacturing the photoelectric conversion elements of Comparative Examples 7 to 12 were respectively the same as those in Comparative Examples 1 to 6, except that a UV-blocking film (KU-1000100 made by KING WORKS Co., Ltd., transmittance of light having a wavelength of 370 nm or less is 1% or less) was attached to the glass substrate of the negative electrode.

### (Evaluation of Photoelectric Conversion Efficiency and Open Circuit Voltage)

Current-voltage characteristics of the photoelectric conversion elements of Examples 1 to 6 and Comparative Examples 1 to 12 in the initial state were measured, while simulated sunlight having an illumination of 1000 W/m² was being emitted at room temperature. The photoelectric conversion efficiencies and open circuit voltages of the solar cells were calculated from the obtained current-voltage characteristics. The obtained results of the photoelectric conversion efficiencies and open circuit voltages are shown in Table 2.

### (Production Yield)

A production yield in each of Examples and Comparative Examples was calculated as follows. The obtained results of the production yields are shown in Table 2.
(1) Ten or more of the same elements were manufactured to evaluate a photoelectric conversion efficiency;
(2) after an element, the photoelectric conversion efficiency of which is clearly low due to a short circuit, etc., is removed, the average of the photoelectric conversion efficiencies is calculated; and
(3) assuming that a photoelectric conversion element, the photoelectric conversion efficiency of which is 75% or more of the calculated average, is a good product, a production yield is calculated from the equation: production yield [%] = 100 × the number of good products/the total number.

### (Measurement of Conduction Band Bottom Energy)

In evaluating the conduction band bottom energy of each of an electron extraction layer and an electron transport layer, an ionization potential determined by ultraviolet photoelectron spectroscopy and a bandgap determined by ultraviolet-visible absorption spectrum were used (Reference document: "Electronic Properties of Conjugated Polyelectrolyte Thin Film" written by T. C. Nguyen, J. AM. CHEM. SOC., 2008, 130, 10042-10043). The photoelectron spectrum of each of an electron extraction layer and an electron transport layer was measured by ultraviolet photoelectron spectroscopy, in which He(I) was excited with energy of 21.22 eV, with the use of AXIS Ultra (made by Kratos Analytical Limited) as an ultraviolet photoelectron spectrometer. An ionization potential was evaluated from the cutoff energy on the high binding energy side of the obtained photoelectron spectrum. The measurement of ultraviolet-visible absorption spectra was carried out by using U-4100 made by Hitachi High-Technologies Corp. A bandgap was evaluated from the cutoff on the long wavelength side of the obtained absorption spectrum. A conduction band bottom energy was calculated by deducting the optical bandgap from the ionization potential. The obtained results of the conduction band bottom energy is shown in Table 2. When the conduction band bottom energy of each of the electron extraction layer and the electron transport layer, which were incorporated in the photoelectric conversion element, was measured, it was determined as follows: one side of the cover glass in the photoelectric conversion element was peeled off such that the photoelectric conversion layer was dissolved and removed with o-dichlorobenzene; and then the conduction band bottom energy was calculated by the aforementioned method and by using a substrate with the electron extraction layer that remained on the substrate, while the electron extraction layer was being dug in the film thickness direction by Ar sputtering.

**[Table 2]**

| | EVALUATION RESULTS | | | | | |
|---|---|---|---|---|---|---|
| | PHOTOELECTRIC CONVERSION EFFICIENCY[%] | OPEN CIRCUIT VOLTAGE [V] | PRODUCTION YIELD [%] | CONDUCTION BAND BOTTOM ENERGY ADJUSTMENT LAYER PRESENCE/ABSENCE | CONDUCTION BAND BOTTOM ENERGY OF ELECTRON EXTRACTION LAYER [eV] | OXIDATION-REDUCTION POTENTIAL OF N-TYPE MATERIAL OF PHOTOELECTRIC CONVERSION LAYER [mV] |
| EXAMPLE 1 | 5.01 | 0.77 | >95 | PRESENCE | 2.9 | 1350 |
| EXAMPLE 2 | 5.26 | 0.78 | >95 | PRESENCE | 3.3 | 1350 |
| EXAMPLE 3 | 5.51 | 0.79 | >95 | PRESENCE | 2.9 | 1350 |
| EXAMPLE 4 | 4.79 | 0.77 | >95 | PRESENCE | 2.9 | 1350 |
| EXAMPLE 5 | 5.03 | 0.78 | >95 | PRESENCE | 3.3 | 1350 |
| EXAMPLE 6 | 5.27 | 0.79 | >95 | PRESENCE | 2.9 | 1350 |
| COMPARATIVE EXAMPLE 1 | 5.03 | 0.80 | >95 | ABSENCE | 4.0 | 1350 |
| COMPARATIVE EXAMPLE 2 | 3.17 | 0.61 | >95 | ABSENCE | 4.4 | 1350 |
| COMPARATIVE EXAMPLE 3 | 5.02 | 0.81 | >95 | ABSENCE | 4.0 | 1350 |
| COMPARATIVE EXAMPLE 4 | 5.27 | 0.77 | 65 | ABSENCE | 3.7 | 1350 |
| COMPARATIVE EXAMPLE 5 | 5.19 | 0.80 | >95 | PRESENCE | 4.0 | 1350 |
| COMPARATIVE EXAMPLE 6 | 5.20 | 0.81 | >95 | PRESENCE | 4.0 | 1350 |
| COMPARATIVE EXAMPLE 7 | 2.67 | 0.80 | >95 | ABSENCE | 4.0 | 1350 |
| COMPARATIVE EXAMPLE 8 | 3.03 | 0.61 | >95 | ABSENCE | 4.4 | 1350 |
| COMPARATIVE EXAMPLE 9 | 4.80 | 0.81 | >95 | ABSENCE | 4.0 | 1350 |
| COMPARATIVE EXAMPLE 10 | 5.03 | 0.76 | 65 | ABSENCE | 3.7 | 1350 |
| COMPARATIVE EXAMPLE 11 | 4.96 | 0.80 | >95 | PRESENCE | 4.0 | 1350 |
| COMPARATIVE EXAMPLE 12 | 4.97 | 0.81 | >95 | PRESENCE | 4.0 | 1350 |

### (Identification of Oxidation-Reduction Potential of N-type Material of Photoelectric Conversion Layer)

Identification of the oxidation-reduction potential of the n-type material of a photoelectric conversion layer was carried out as follows, with reference to "Electochemical Methods: Fundamentals and Applications" written by A.J. Bard.

An o-dichlorobenzene 0.1M solution of tetrabutylammonium perchlorate was manufactured, and 4 mg of ferrocene was added, as an internal reference substance, to per 50 mL of the solution to prepare a measuring solution. To 2 mL of this solution, 1.5 mg of a fullerene derivative was added, and an oxidation-reduction potential was measured at a sweep rate of 20 mV/s by using a potentiostat galvanostat (electrochemical analyzer: model 630A made by ALS). A first reduction potential was determined as the average of the first reduction peak and its oxidation peak, based on the oxidation/reduction potential (Fc/Fc⁺) of the ferrocene added as an internal reference substance. Herein, the oxidation-reduction potential of the n-type material of the photoelectric conversion layer incorporated in the photoelectric conversion element was identified as follows: after one side of the cover glass in the photoelectric conversion element was peeled off and the photoelectric conversion layer was dissolved with o-dichlorobenzene, tetrabutylammonium perchlorate and 4 mg of ferrocene, as an internal reference substance, were added to per 50 mL of the solvent to prepare a measuring solution. With the use of the obtained solution, an oxidation-reduction potential was measured at a sweep rate of 20 mV/s by using a potentiostat galvanostat (electrochemical analyzer: model 630A made by ALS). The first reduction potential was determined as the average of the first reduction peak and its oxidation peak, based on the oxidation/reduction potential (Fc/Fc⁺) of the ferrocene added as an internal reference substance.

### (Light Resistance Test)

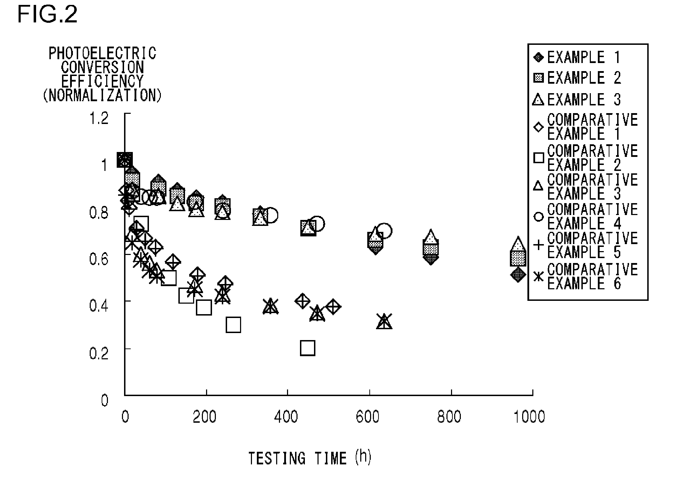

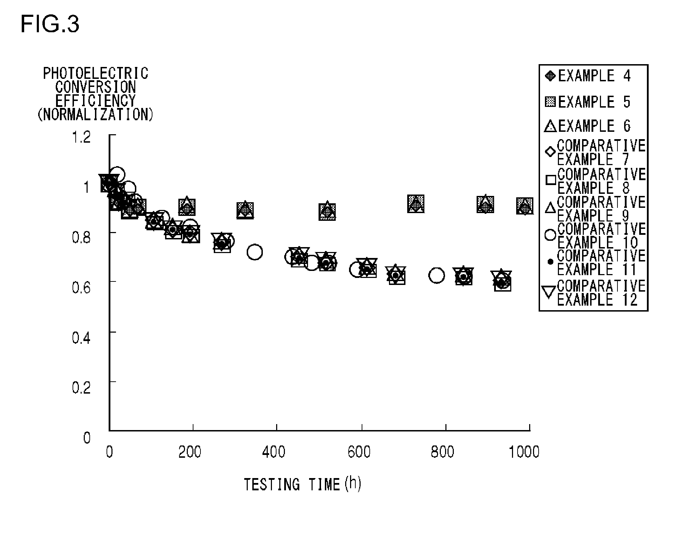

Fig. 2 is a graph showing light resistance in each of the photoelectric conversion elements of Examples 1 to 3 and Comparative Examples 1 to 6. Fig. 3 is a graph showing light resistance in each of the photoelectric conversion elements of Examples 4 to 6 and Comparative Examples 7 to 12. A light resistance test was carried out by using a light resistance tester (SUNTES-XLS made by ATLAS MATERIAL TESTING SOLUTIONS) and in the following way: the illuminance of a xenon lamp was adjusted with an ultraviolet radiometer (MS-212A made by EKO Instruments) such that an amount of ultraviolet rays having a wavelength of 315 to 400 nm becomes 45 W/m²; and then a sample was put in and continuously irradiated with the rays at ambient temperature of 40°C for 1000 hours. Photoelectric conversion efficiencies after predetermined periods of time were plotted in Figs. 2 and 3, based on the photoelectric conversion efficiency in the initial state.

As shown in Fig. 2, in each of the photoelectric conversion elements of Examples 1 to 3 in which a conduction band bottom energy adjustment layer, having a large effect of reducing the conduction band bottom energy of an electron extraction layer, was provided, a production yield is improved, compared with the photoelectric conversion element of Comparative Example 4 in which a cesium carbonate layer was provided as an electron transport layer; and a decrease in the photoelectric conversion efficiency is greatly suppressed, compared with both the photoelectric conversion elements of Comparative Examples 1 to 3 in which a conduction band bottom energy adjustment layer was not provided, and those of Comparative Examples 5 and 6 in which an effect of reducing the conduction band bottom energy of an electron extraction layer was small. Further, in each of the photoelectric conversion elements of Examples of 4 to 6 in which a UV-blocking film was attached, it has been confirmed that: a decrease in the photoelectric conversion efficiency is greatly suppressed, compared with both the photoelectric conversion elements of Comparative Examples 7 to 10 in which the same UV-blocking film was attached but a conduction band bottom energy adjustment layer was not provided, and those of Comparative Examples 11 and 12 in which an effect of reducing the conduction band bottom energy of an electron extraction layer was small; and after the continuous emission for 40 hours, the photoelectric conversion efficiency is not hardly decreased such that 90% or more of the photoelectric conversion efficiency in the initial state is maintained.

The present invention should not be limited to the aforementioned embodiments, and various modifications, such as design modifications, can be made with respect to the embodiments based on the knowledge of those skilled in the art, and an embodiment with such a modification can be encompassed within the scope of the present invention.

For example, in the photoelectric conversion element 10 according to an embodiment, the electron extraction layer 90, including both the electron transport layer 40 and the conduction band bottom energy adjustment layer 50, is provided between the first electrode 30 and the photoelectric conversion layer 60, and the hole transport layer 70 is provided between the photoelectric conversion layer 60 and the second electrode 80; however, the position of the hole transport layer 70 and that of the electron extraction layer 90 can be replaced with each other. When the hole transport layer 70 is provided in a region between the first electrode 30 and the photoelectric conversion layer 60 and the electron extraction layer 90 is provided in a region between the second electrode 80 and the photoelectric conversion layer 60, the first electrode 30 serves as a positive electrode and the second electrode 80 as a negative electrode. In this case, the conduction band bottom energy adjustment layer 50, the electron transport layer 40, and the second electrode 80 are provided in this order on the surface of the photoelectric conversion layer 60 opposite to its light-receiving surface.

### Reference Numerals

- 10: PHOTOELECTRIC CONVERSION ELEMENT

- 20: SUBSTRATE
- 30: FIRST ELECTRODE
- 40: ELECTRON TRANSPORT LAYER,
- 50: CONDUCTION BAND BOTTOM ENERGY ADJUSTMENT LAYER
- 60: PHOTOELECTRIC CONVERSION LAYER
- 70: HOLE TRANSPORT LAYER
- 80: SECOND ELECTRODE
- 90: ELECTRON EXTRACTION LAYER

### Industrial Applicability

The present invention can be applied to fields related to a photoelectric conversion element that converts light energy into electrical energy by photoelectric conversion.

## Claims

1. A photoelectric conversion element comprising:
at least a photoelectric conversion layer;
an electron extraction electrode provided on one major surface side of the photoelectric conversion layer;
a hole extraction electrode provided on the other major surface side of the photoelectric conversion layer; and
an electron extraction layer that is provided between the photoelectric conversion layer and the electron extraction electrode and includes at least an electron transport layer, wherein
the photoelectric conversion element further comprises, between the photoelectric conversion layer and the electron transport layer, a conduction band bottom energy adjustment layer configured to reduce conduction band bottom energy of the electron extraction layer to energy lower than conduction band bottom energy of the electron transport layer.

2. The photoelectric conversion element according to claim 1, wherein
when xenon lamp light, the ultraviolet ray intensity of which is adjusted to be equivalent to 1 Sun, is continuously emitted at ambient temperature of 40°C for 1000 hours from the initial state where the light is not emitted, a decrease rate of photoelectric conversion efficiency is 10% or less.

3. The photoelectric conversion element according to claim 1 or claim 2, wherein
the conduction band bottom energy adjustment layer contains a cesium compound.

4. The photoelectric conversion element according to any one of claims 1 to 3, wherein
the electron transport layer contains a substance represented by the following chemical formula and a reactant obtained from one or more substances represented by the following chemical formula:
M(X)ₐ (1)
wherein M is a metal or alloy selected from the group consisting of alkali metals, alkaline earth metals, group 2B and 3B metals, and transition metals; X is selected from oxygen, a halogen, carboxylate group, alkoxy group, alkyl group, and acetonate group represented by the following chemical formula; and a is a positive integer determined in accordance with the valence of M: 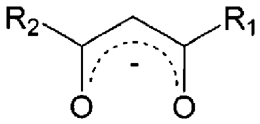 wherein R₁ and R₂ are selected from hydrogen, a C₁₋₂₀ linear or branched alkyl group, and C₁₋₂₀ linear or branched alkoxy group, and R₁ and R₂ may or may not be the same as each other.

5. The photoelectric conversion element according to any one of claims 1 to 4, wherein
the electron transport layer contains one or more metal compounds and reactants thereof, the metal compounds being selected from the group consisting of zinc acetate, magnesium acetate, aluminum acetylacetonate, aluminum chloride, gallium acetylacetonate, gallium chloride, zinc acetylacetonate, zinc chloride, diethylzinc, and ZnMgO.

6. The photoelectric conversion element according to any one of claims 1 to 5, wherein
the photoelectric conversion layer contains a fullerene derivative having a first reduction potential of 1160 mV (vs Fc/Fc⁺) or more.

7. The photoelectric conversion element according to claim 6, wherein the fullerene derivative is ICBA (Indene-C60 bisadduct).

8. A method of manufacturing a photoelectric conversion element having a pair of electrodes, a photoelectric conversion layer provided between the pair of electrodes, an electron transport layer provided between one of the electrodes and the photoelectric conversion layer, and a conduction band bottom energy adjustment layer interposed between the photoelectric conversion layer and the electron transport layer, the method comprising:
forming the electron transport layer by heating a film, which has been formed by applying a solution containing a substance represented by the following chemical formula (1-1), to a temperature (t₁) of 100°C ≤ t₁ ≤ 150°C; and
forming the conduction band bottom energy adjustment layer containing a cesium compound:
Zn(X)₂ (1-1)
wherein X is selected from a halogen, carboxylate group, alkoxy group, alkyl group, and acetonate group represented by the following formula: 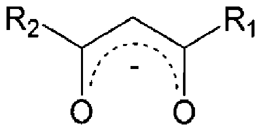 wherein R₁ and R₂ are selected from hydrogen, a C₁₋₂₀ linear or branched alkyl group, and C₁₋₂₀ linear or branched alkoxy group, and R₁ and R₂ may or may not be the same as each other.

9. A method of manufacturing a photoelectric conversion element having a pair of electrodes, a photoelectric conversion layer provided between the pair of electrodes, an electron transport layer provided between one of the electrodes and the photoelectric conversion layer, and a conduction band bottom energy adjustment layer interposed between the photoelectric conversion layer and the electron transport layer, the method comprising:
forming the electron transport layer by heating a film, which has been formed by applying a solution containing substances represented by the following chemical formulae (1-1) and (1-2), to a temperature of 300°C or higher; and
forming the conduction band bottom energy adjustment layer containing a cesium compound:
Zn(X)₂ (1-1)
wherein X is selected from a halogen, carboxylate group, alkoxy group, alkyl group, and acetonate group represented by the following formula: 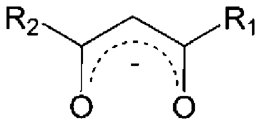 wherein R₁ and R₂ are selected from hydrogen, a C₁₋₂₀ linear or branched alkyl group, and C₁₋₂₀ linear or branched alkoxy group, and R₁ and R₂ may or may not be the same as each other,
Mg(X)₂ (1-2)
wherein X is selected from a halogen, carboxylate group, alkoxy group, alkyl group, and acetonate group represented by the following formula: 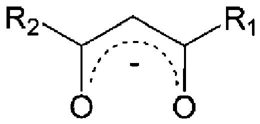 wherein R₁ and R₂ are selected from hydrogen, a C₁₋₂₀ linear or branched alkyl group, and C₁₋₂₀ linear or branched alkoxy group, and R₁ and R₂ may or may not be the same as each other.
